# EUROPÄISCHE PATENTSCHRIFT

(11) **EP 1 616 540 B1**
(45) Veröffentlichungstag und Bekanntmachung des Hinweises auf die Patenterteilung: **14.11.2007**
(21) Anmeldenummer: 04016593.8
(22) Anmeldetag: 14.07.2004
(51) Int. Cl.: A61F 2/46, A61B 19/00, A61B 17/72

(54) **Positionierungssystem mit kannuliertem Implantat**
Positioning system with cannulated implant
Système de positionnement avec un implant cannelé

(43) Veröffentlichungstag der Anmeldung: 18.01.2006
(73) Patentinhaber: BrainLAB AG, 85551 Kirchheim/Heimstetten (DE)
(72) Erfinder: Plassky, Norman, 99099 Erfurt (DE); Tuma, Gregor, 81675 München (DE)
(74) Vertreter: Schwabe - Sandmair - Marx

(56) Entgegenhaltungen:
- DE-A- 19 639 615
- US-A- 5 116 377
- US-A- 5 514 137
- US-A- 5 624 443
- US-A1- 2002 095 217

## Beschreibung

Die vorliegende Erfindung betrifft ein Positionierungssystem zum Setzen eines Implantats in einen Hohlraum eines Knochens. Insbesondere betrifft die vorliegende Erfindung ein Positionierungssystem für ein Revisionsimplantat.

Es existieren derzeit unterschiedliche Prozeduren für das Setzen von Implantaten oder das Aufbauen von knochenähnlicher Struktur unter Verwendung von Zement und anderen Füllmaterialien. Meist wird Knochenzement in einen freien oder freigelegten Knochenkanal, beispielsweise einen Oberschenkel-Knochenhohlraum, eingefüllt, nachdem das alte Implantat entfernt worden ist. Dies kann mit Hilfe von kannulierten Stangen geschehen, oder durch ein anderweitiges Einbringen des Zements in den Knochenhohlraum. Manchmal werden Probeimplantate verwendet, um eine Form gemäß dem echten Implantat auszubilden, welches dann eingebracht wird, nachdem der Zement ausgehärtet ist. Bei anderen Methoden wird ein Stift in den Zement eingesetzt und als Trajektorie für das Ausräumen eines neuen Kanals bzw. Hohlraums verwendet. Kanülen, die nach ihrer Verwendung wieder entfernt werden, werden z.B. auch zum Wiederaufbau von Wirbelknochen verwendet.

Gängige Methoden umfassen zudem das Einpressen eines Implantats oder Revisionsimplantats in den Knochenhohlraum, der beispielsweise mit einem navigierten Werkzeug ausgeräumt sein kann. Dabei ist das Implantat meist geringfügig größer ausgestaltet als der Hohlraum, in den es eingebracht werden soll, damit eine Art "Presspassung" erzielt werden kann, die das Implantat im Hohlraum sicher hält. In anderen Fällen wird das Implantat mit Haftstoffen, Füllstoffen oder Zement ummantelt, die sich dann im Hohlraum verteilen sollen. In beiden oben genannten Fällen kann keine gute Genauigkeit bei der Positionierung des Implantats erzielt werden, im letzteren Fall ist nicht gewährleistet, dass alle verbleibenden Hohlräume sicher ausgefüllt werden.

Solche bisher verwendeten Systeme und Prozeduren sind beispielsweise aus dem nachfolgenden Veröffentlichungen bekannt: US 6,142,998; US 5,171,288; US 5,849,014;US 5,192,283; US 6,270,502; US 5,624,443; US 2003/0083662 A1 und US 2003/0105468 A1.

Die Merkmale des Oberbegriffs des Anspruchs 1 sind aus der US-A-2002/0095217 bekannt.

Es ist die Aufgabe der vorliegenden Erfindung, ein Positionierungssystem zum Setzen eines Implantats in einen Hohlraum eines Knochens zu schaffen, welches eine sehr genaue Positionierung des Implantats ermöglicht. Insbesondere soll auch gewährleistet werden, dass hohe Kräfte auf den umgebenden Knochen vermieden werden, und speziell soll das System eine vollständige Auffüllung des Knochenhohlraums gestatten.

Diese Aufgabe wird erfindungsgemäß durch ein Positionierungssystem gemäß dem Anspruch 1 gelöst. Die Unteransprüche definieren bevorzugte Ausführungsformen der Erfindung.

Gemäß der vorliegenden Erfindung weist das Positionierungssystem ein kannulliertes Implantat auf, das im Implantierungsbereich im Wesentlichen kleiner ist als der Hohlraum, sowie eine Positionierungsvorrichtung, die das Implantat in einstellbarer Position im Hohlraum halten kann. Mit anderen Worten wird eine Vorrichtung bereitgestellt, welche ein Stellsystem für das Implantat aufweist, das dafür sorgt, dass das Implantat genau in der eventuell durch pre-operative Planung nach biomechanischen, anatomischen und technischen Gesichtspunkten richtigen Stellung im Knochenhohlraum platziert und gehalten wird, bevor über die Kanülen im Implantat das Füll-, Haft-, oder Zementmaterial in den Hohlraum eingebracht wird. Der Begriff "im Wesentlichen kleiner als der Hohlraum" bedeutet, dass die Außenabmessungen des Implantats im Wesentlichen kleiner sind als die Innenabmessungen des Hohlraums bzw. diese Innenabmessungen nicht an einer wesentlichen Anzahl von Stellen und über eine wesentliche Länge im Implantierungsbereich überschreiten. Wenn dem so ist, kann das Implantat auch nach dem Einsetzen in den Knochenhohlraum noch genau positioniert werden.

Der Vorteil des erfindungsgemäßen Systems liegt damit insbesondere darin, dass einerseits Belastungen der Knochenstruktur von innen durch das Implantat weitgehendst vermieden werden können und andererseits wegen der Kanülenbefüllung sichergestellt werden kann, dass alle Hohlräume im Knochen ausgefüllt werden. Natürlich wird durch die erfindungsgemäße Einstellbarkeit der Position auch die optimale Endposition des Implantats gewährleistet. Die Erfindung ermöglicht insgesamt das Setzen von Implantaten mit weniger Schritten, da keine Probeimplantate gesetzt werden müssen und sich navigierte oder auch nicht navigierte Ausräumprozesse erübrigen.

Bei einer bevorzugten Ausführungsform der Erfindung ist dem Implantat eine Referenzanordnung zugeordnet, welche die Ortung und Verfolgung des Implantats und dessen Relativposition gegenüber dem Hohlraum mittels eines chirurgischen Navigationssystems gestattet. Die direkte Navigation des Implantats führt zu einer besseren und unmittelbareren Positionskontrolle als bisher vorgeschlagene, navigierte Hohlraum-Ausräumverfahren. Bekannte Navigationssysteme können verwendet werden; es können aktive oder passive Systeme (mit abstrahlenden oder reflektierenden Referenzmarkem und Kamera-Trackingsystemen) sein, und es kommt sowohl die optische als auch die magnetische oder elektromagnetische Navigation in Frage. Ein verwendbares Navigationssystem ist beispielsweise aus der DE 196 39 615 C2 bekannt. Die Überwachung und Steuerung der Positionierung muss nicht unbedingt unter Zuhilfenahme eines Navigationssystems erfolgen sondern kann auch z.B. mit Hilfe intra-operativen Röntgens durchgeführt werden.

Die Positionierungsvorrichtung kann an einer Seite positionsfest gegenüber dem Knochen angeordnet sein und an der anderen Seite das Implantat halten. Sie ist vorteilhafterweise grundsätzlich zwischen dem Knochen und dem Implantat angeordnet, damit die Stellbewegungen sich unmittelbar auf die Positionierung gegenüber diesem Knochen (Knochenhohlraum) auswirken. Natürlich kann in einer solchen Ausgestaltung aber auch eine am Knochen angebrachte Halteeinrichtung verwendet werden, mittels der die Positionierungseinrichtung positionsfest gegenüber dem Knochen angeordnet wird. Eine solche Halteeinrichtung kann eine Art Klammereinrichtung sein.

Die Positionierungsvorrichtung wird in speziellen Fällen mechanische oder elektromechanische Stellvorrichtungen aufweisen, die eine Längs- und/oder Querverschiebung und/oder eine Drehung des Implantats im Hohlraum gestatten. Solche Stellvorrichtungen können von bekannter Art sein, beispielsweise Gelenkvorrichtungen, Spindeltriebe, oder andere bekannte winkel- und längenverstellbare Einrichtungen, die sich vorzugsweise feststellen lassen, wenn das Implantat die gewünschte Position eingenommen hat.

Auch dem Knochen oder der Halteeinrichtung kann eine Referenzanordnung zugeordnet werden, welche die Ortung und Verfolgung des Knochens bzw. der Halteeinrichtung mittels eines chirurgischen Navigationssystems, insbesondere desselben Navigationssystems, welches das Implantat ortet und verfolgt, gestattet. Damit wird eine gute relative Navigation des Implantats gegenüber dem Knochen, also direkt gegenüber dem Teil, das den Hohlraum aufweist, ermöglicht.

Die Referenzanordnungen (am Implantat oder am Knochen bzw. an der Halteeinrichtung) können fest oder feststellbar beweglich angeordnet sein, im letzter Fall lässt sich die Sichtbarkeit für ein optisches Navigationssystem noch durch eine Verstellung der Referenzanordnung verbessern. Bei einer bevorzugten Ausführungsform bilden Referenzanordnung und Implantat eine vorkalibrierte Einheit, deren Geometrie im Navigationssystem bekannt und eindeutig zuordnungsfähig ist.

Das Implantat kann ein kannulliertes Permanent- bzw. Revisionsimplantat mit einem Einlass und Umfangsauslässen für eine Befestigungsmasse sein, wobei der Einlass ein separat vorgesehener Einlass ist oder durch die Schnittstelle zur Aufnahme des Implantatkopfes führt.

Die Erfindung wird im Weiteren anhand einer bevorzugten Ausführungsform näher erläutert. Die hierin beschriebenen Merkmale können natürlich auch verfahrensmäßig und computergestützt umgesetzt werden, und sie sind einzeln oder in jedweder Kombination im Rahmen der Erfindung anwendbar. Im Folgenden wird auf die beiliegenden Zeichnungen Bezug genommen; es zeigen:
- Figur 1: ein erfindungsgemäßes Positionierungssystem im Einsatz beim Setzen eines Oberschenkelknochen-Implantats;
- Figur 2: ein mit dem erfindungsgemäßen System verwendbares Implantat;
- Figur 3: den Sitz des Implantats im Oberschenkelknochen nach dem Setzvorgang; und
- Figur 4: das in den Knochenhohlraum eingesetzte Implantat in einer Sicht auf den Hohlraumeingang.

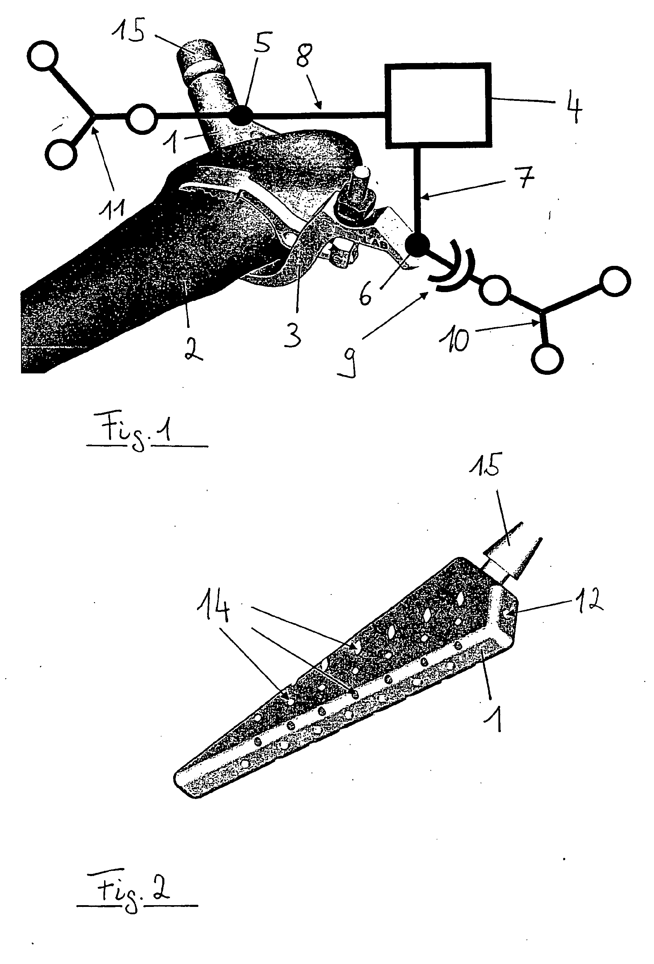

In den Figuren sind gleiche Teile mit gleichen Bezugszeichen versehen. Das erfindungsgemäß verwendbare Implantat trägt in allen Figuren das Bezugszeichen 1. Das Implantat 1 ist dazu bestimmt, in einen Hohlraum 13 (Figur 4) eines Oberschenkelknochens 2 (Figuren 1, 3 und 4) eingesetzt zu werden, und es ist in Figur 2 näher dargestellt. Es weist einen sich nach vorne zum distalen Ende hin verjüngenden Implantatkörper auf, der an seinem Umfang Auslässe 14 für ein Fluid aufweist (Füllmaterial, z.B. Knochenzement), welches nach dem Setzen des Implantats die Hohlräume zwischen dem Implantat 1 und den inneren Knochenwandungen ausfüllen sowie das Implantat 1 befestigen soll. Eingespritzt wird das Fluid über die Einlassöffnung 12 des Implantats 1, die in den Figuren 2 und 4 zu sehen ist. Eine Schnittstelle zur Aufnahme eines Implantatkopfes ist mit dem Bezugszeichen 15 versehen. Es sind auch Ausführungsformen denkbar, bei denen die Einlassöffnung durch diese Schnittstelle führt.

Um das Implantat 1 positionsgenau setzen zu können, wird die erfindungsgemäße Einstellvorrichtung verwendet, die in Figur 1 schematisch gezeigt und mit dem Bezugszeichen 4 versehen ist. Die Einstellvorrichtung 4 weist, wie oben schon angedeutet, Winkelstellvorrichtungen, Längenstellvorrichtungen oder Drehstellvorrichtungen auf, oder eine Kombination aus solchen Stellvorrichtungen, und sie ist mit einem Arm 7 positionsfest gegenüber dem Knochen 2 angeordnet, in diesem Fall dadurch, dass sie mit dem Arm 7 am Feststellungspunkt 6 an einer Klammer 3 sitzt, die am Knochen 2 angeklammert ist. Der Knochen 2 wird in seiner Position durch ein nicht dargestelltes Navigationssystem geortet und positionell verfolgt, und zwar über den Referenzstern 10, der wiederum positionsverstellbar über den Adapter 9 an der Klammer 3 angeordnet ist. Die Positionsverstellung für den Referenzstern 10 über den Adapter 9 kann erfolgen, um den Stern 10 in dem Navigationssystem, das heißt für die Navigationskameras, besser sichtbar zu machen und ihn in einer gut sichtbaren Position fest zu stellen.

Auf ihrer anderen Seite weist die Positionierungsvorrichtung 4 den Stellarm 8 auf, der an der Festlegungsstelle 5 mit dem Implantat fest verbunden ist. Durch die Stellbewegungen der Positionierungsvorrichtung 4 wird das Implantat 1 längs oder im Winkel verschoben oder gedreht. Einstellbar sind die Eindringtiefe, die Varus/Valgus-Rotation, die femorale Antetorsion, der mediale/laterale Shift oder der Anterior/Posterior-Kippwinkel. Grundsätzlich ist die Verstellbarkeit in allen Freiheitsgraden denkbar. Entsprechende Stellvorrichtungen sind bekannt und müssen hier nicht separat beschrieben werden.

Ebenfalls starr mit dem Implantat 1 ist der Referenzstern 11 verbunden, der die Ortung und Positionsverfolgung für das Implantat 1 im Navigationssystem ermöglicht. Im vorliegenden Fall wird die starre Verbindung gezeigt, da es sich gemäß einer bevorzugten Ausführungsform der Erfindung hier um eine vorkalibrierte Einheit aus Referenzstern 11 und Implantat 1 handelt, deren Geometrie charakteristisch und im Navigationssystem bekannt ist. Es ist anzumerken, dass die Verwendung solcher vorkalibrierten Implantate im Rahmen der Erfindung nicht unbedingt notwendig ist; es ist auch möglich, nicht vorkalibrierte Implantate zu verwenden, einen Referenzstern oder ein anderes Referenzmittel daran anzubringen und erst im Laufe der Verwendung eine Kalibrierung durchzuführen. In einem solchen Fall kann - wie beim Referenzstern 10 - auch ein beweglich, aber feststellbar angeordneter Referenzstern am Implantat angeordnet werden.

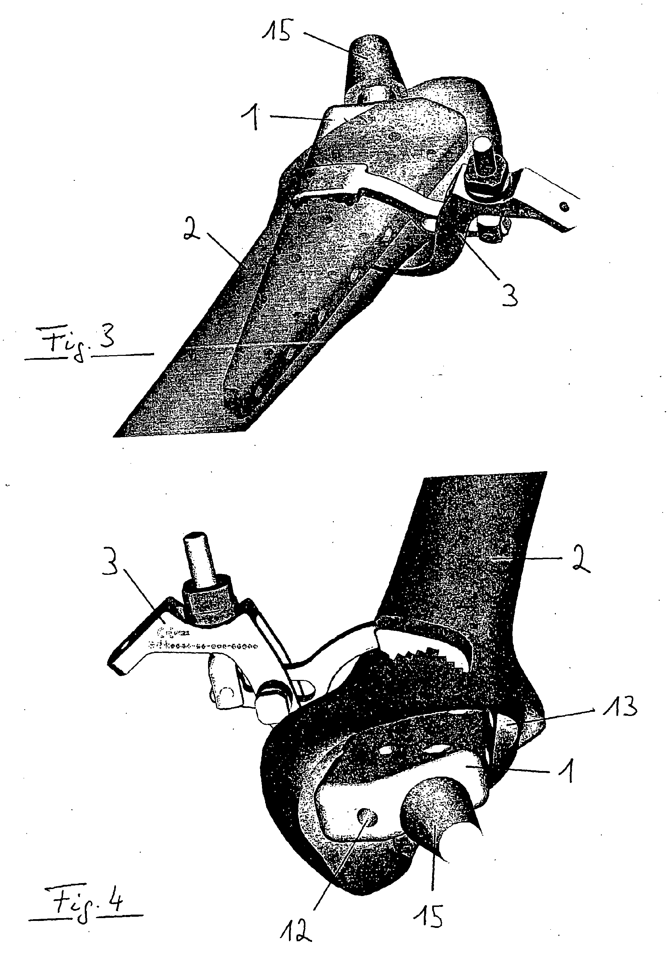

In Figur 3 ist zu sehen, wie das Implantat innerhalb des Knochenhohlraums zu liegen kommt, dazu ist die Knochenwandung teilweise durchsichtig dargestellt worden. Wie insbesondere aus der Figur 4 hervorgeht, ist das Implantat in seinen äußerem Abmessungen kleiner als der Hohlraum im Inneren des Knochens 13, es kann also eingesetzt und optimal positioniert werden, ohne dass die Knochenstruktur belastet oder gar beschädigt wird. Die verbleibenden Hohlräume werden dann bei optimaler Positionierung mittels des Einbringens des Füllmaterials (Knochenzement) durch Einlass 12 und die Auslässe 14 aufgefüllt.

Im Folgenden wird noch die Verwendung des erfindungsgemäßen Positionierungssystems anhand eines speziellen Vorgangs beschrieben, bei dem ein Implantat 1 in den Hohlraum 13 des Knochens 2 eingesetzt wird. Als Vorbedingung hat der Patient einen offenen oder ausgeräumten, d.h. mit dem Hohlraum 13 versehenen Oberschenkelkanal. Dieser Oberschenkelkanal bzw. der Hohlraum 13 kann das Resultat eines schon entfernten, alten Implantats sein; er kann aber auch, z.B. zum Entfernen eines krebskranken Knochenmaterials, freigeräumt worden sein. Für die Durchführung und zur Unterstützung der Prozedur steht ein Navigationssystem zur Verfügung, und zunächst wird der Referenzstern 10 mit Hilfe der Klammer 3 am Knochen 2 befestigt und in gut sichtbarer Position für das Kamerasystem der Navigations-Trackingeinrichtung mit Hilfe des Adapters 9 festgestellt. Hier ist anzumerken, dass ein Referenzstern 10 auch unmittelbar am Knochen z.B. am distalen Ende des Knochens angebracht werden könnte.

Mit Hilfe des Referenzsterns 10 wird der Knochen im Navigationssystem registriert, also positionell zugeordnet.

Als nächstes wird an dem Implantat der Referenzstern 11 angeordnet. Falls es sich bei dem System aus Referenzstern 11 und Implantat 1 um eine dem Navigationssystem bekannte, vorkalibrierte Einheit handelt, muss diese nicht mehr dort registriert werden, in sonstigen Fällen ist auch hier eine Registrierung durchzuführen. Hierauf kann das Implantat mit dem Referenzstern 11 nun grob positioniert in den Hohlraum 13 des Knochens 2 eingesetzt werden, worauf die Positionierungsvorrichtung 4 sowohl am Implantat 1 (über den Stellarm 8) als auch an der Klammer 3 (über den Arm 7) angebracht wird.

Nunmehr kann mit Hilfe der Positionsinformationen über Knochen und Implantat, die von dem Navigationssystem erhalten werden, die Position und Ausrichtung des Implantats 1 im Hohlraum 13, also gegenüber dem Knochen 2, durch die Stellbewegungen der Positionsvorrichtung 4 so lange fein eingestellt werden, bis sich das Implantat genau in der richtigen Position befindet, wo es dann fest gestellt wird. Dabei kann die richtige Position beispielsweise mit der Position abgeglichen werden, die vorab durch Planung im Navigationssystem als optimale Position bestimmt wurde. Wenn im Navigationssystem Daten über den Knochen und über die Geometrie des Hohlraums vorhanden sind, lässt sich die Richtigkeit der Positionierung auch für diejenigen Stellen feststellen, die von außen nach dem Einbringen des Implantats nicht mehr sichtbar sind, und entsprechende korrigierende Stellmaßnahmen können getroffen werden.

Wenn das Implantat in der richtigen Position im Hohlraum 13 sitzt, wird die Positionierungseinrichtung 4 fixiert, und es erfolgt das Auffüllen des verbleibenden Hohlraums zwischen Knochen und Implantat mit dem Füllmaterial (Knochenzement) durch den Einlass 12 des Implantats und die Auslässe 14, wobei durch die Anordnung der Auslässe 14 sichergestellt werden kann, dass der gesamte restliche Hohlraum ausgefüllt wird.

Wenn dann das Füllmaterial ausgehärtet ist, können alle Werkzeuge entfernt werden.

## Patentansprüche

1. Positionierungssystem zum Setzen eines Implantats (1) in einen Hohlraum (13) eines Knochens (2), mit einem kannulierten Implantat (1) und mit einer Positionierungsvorrichtung (4), die das Implantat (1) in einstellbarer Position im Hohlraum (13) halten kann, **dadurch gekennzeichnet, dass**
- dem Implantat (1) eine Referenzanordnung (11) zugeordnet ist, welche die Ortung und Verfolgung des Implantats (1) und dessen Relativposition gegenüber dem Hohlraum (13) mittels eines chirurgischen Navigationssystems gestattet, und dass
- dem Knochen (2) eine Referenzanordnung (10) zugeordnet ist, welche die Ortung und Verfolgung des Knochens (2) mittels des chirurgischen Navigationssystems gestattet, wobei
- die Positionierungsvorrichtung (4) eine elektromechanische Stellvorrichtung aufweist, die eine Längs- und/oder Querverschiebung und/oder eine Drehung des Implantats (1) im Hohlraum (13) gestattet.

2. Positionierungssystem nach Anspruch 1 oder 2, **dadurch gekennzeichnet, dass** die Positionierungsvorrichtung (4) an einer Seite positionsfest gegenüber dem Knochen angeordnet ist und an der anderen Seite das Implantat hält.

3. Positionierungssystem nach Anspruch 1 oder 2, **dadurch gekennzeichnet, dass** die Positionierungsvorrichtung (4) mittels einer am Knochen (2) angebrachten Halteeinrichtung, insbesondere einer Klammer (3) positionsfest gegenüber dem Knochen angeordnet ist.

4. Positionierungssystem nach einem der Ansprüche 1 bis 3, **dadurch gekennzeichnet, dass** die Referenzanordnung (11) fest oder feststellbar beweglich am Implantat (1) angeordnet ist.

5. Positionierungssystem nach einem der Ansprüche 2 bis 4, **dadurch gekennzeichnet, dass** die Referenzanordnung (11) fest an einem für das Navigationssystem vorkalibrierten Implantat angeordnet ist.

6. Positionierungssystem nach einem der Ansprüche 1 bis 5, **dadurch gekennzeichnet, dass** die Referenzanordnung (10) fest oder feststellbar beweglich an der Halteeinrichtung (3) angeordnet ist.

7. Positionierungssystem nach einem der Ansprüche 1 bis 6, **dadurch gekennzeichnet, dass** das Implantat ein kannulliertes Implantat (1) mit einem Einlass (12) und Umfangsauslässen (14) für eine Befestigungsmasse ist, wobei der Einlass (12) ein separat vorgesehener Einlass ist oder durch die Schnittstelle zur Aufnahme des Implantatkopfes (15) führt.

8. Positionierungssystem nach Anspruch 1, **dadurch gekennzeichnet, dass** es die Ortung und Verfolgung des Implantats (1) und dessen Relativposition gegenüber dem Hohlraum (13) mittels intra-operativer Röntgenkontrolle gestattet.

## Claims

1. A positioning system for placing an implant (1) into a cavity (13) of a bone (2), comprising a cannulated implant (1) and a positioning device (4) which can hold the implant (1) in a settable position in the cavity (13), **characterised in that**:
- a reference array (11) is assigned to the implant (1), which allows the implant (1) and its relative position with respect to the cavity (13) to be localised and tracked by means of a surgical navigation system; and **in that**
- a reference array (10) is assigned to the bone (2), which allows the bone (2) to be localised and tracked by means of the surgical navigation system; wherein
- the positioning device (4) comprises an electromechanical setting device which allows the implant (1) to be longitudinally and/or transversely shifted and/or rotated in the cavity (13).

2. The positioning system according to claim 1, **characterised in that** the positioning device (4) is arranged positionally fixed with respect to the bone on one side, and holds the implant on the other side.

3. The positioning system according to claim 1 or 2, **characterised in that** the positioning device (4) is arranged positionally fixed with respect to the bone by means of a holding means, in particular a clamp (3), attached to the bone (2).

4. The positioning system according to any one of claims 1 to 3, **characterised in that** the reference array (11) is arranged fixedly on the implant (1) or is arranged on the implant (1) such that it can be moved and fixed.

5. The positioning system according to any one of claims 2 to 4, **characterised in that** the reference array (11) is arranged fixedly on an implant which is pre-calibrated for the navigation system.

6. The positioning system according to any one of claims 1 to 5, **characterised in that** the reference array (10) is arranged fixedly on the holding means (3) or is arranged on the holding means (3) such that it can be moved and fixed.

7. The positioning system according to any one of claims 1 to 6, **characterised in that** the implant is a cannulated implant (1) comprising an inlet (12) and peripheral outlets (14) for a fixing mass, wherein the inlet (12) is a separately provided inlet or leads through the interface for receiving the implant head (15).

8. The positioning system according to claim 1, **characterised in that** it allows the implant (1) and its relative position with respect to the cavity (13) to be localised and tracked by means of intra-operative x-ray controlling.

## Revendications

1. Système de positionnement pour placer un implant (1) dans une cavité (13) d'un os (2), avec un implant cannelé (1) et avec un dispositif de positionnement (4) qui peut maintenir l'implant (1) dans la cavité (13), dans une position réglable, **caractérisé en ce que**
- à l'implant (1) est associé un agencement de référence (11) qui permet la localisation et la poursuite de l'implant (1) et de sa position relative par rapport à la cavité (13) au moyen d'un système de navigation chirurgical, et **en ce que**
- à l'os (2) est associé un agencement de référence (10) qui permet la localisation et la poursuite de l'os (2) au moyen du système de navigation chirurgical,
- le dispositif de positionnement (4) comportant un dispositif de réglage électromécanique qui permet une translation longitudinale et/ou transversale et/ou une rotation de l'implant (1) dans la cavité (13).

2. Système de positionnement selon la revendication 1 ou 2, **caractérisé en ce que** le dispositif de positionnement (4) est disposé sur un côté, dans une position fixe par rapport à l'os, et maintient l'implant sur l'autre côté.

3. Système de positionnement selon la revendication 1 ou 2, **caractérisé en ce que** le dispositif de positionnement (4) est disposé dans une position fixe par rapport à l'os, au moyen d'un dispositif de maintien, en particulier une agrafe (3), placé sur l'os (2).

4. Système de positionnement selon l'une des revendications 1 à 3, **caractérisé en ce que** l'agencement de référence (11) est disposé de manière fixe ou déplaçable et fixable sur l'implant (1).

5. Système de positionnement selon l'une des revendications 2 à 4, **caractérisé en ce que** l'agencement de référence (11) est disposé fixe sur un implant précalibré pour le système de navigation.

6. Système de positionnement selon l'une des revendications 1 à 5, **caractérisé en ce que** l'agencement de référence (10) est disposé fixe ou déplaçable et fixable sur le dispositif de maintien (3).

7. Système de positionnement selon l'une des revendications 1 à 6, **caractérisé en ce que** l'implant est un implant cannelé (1) avec une entrée (12) et des sorties périphériques (14) pour une pâte de fixation, l'entrée (12) étant une entrée prévue séparément ou passant par l'interface pour recevoir la tête d'implant (15).

8. Système de positionnement selon la revendication 1, **caractérisé en ce qu'**il permet la localisation et la poursuite de l'implant (1) et de sa position relative par rapport à la cavité (13) au moyen d'un contrôle radiographique en cours d'opération.
